(19) Europäisches Patentamt
European Patent Office
Office européen des brevets

(11) Veröffentlichungsnummer: **0 243 619 B1**

# (12) EUROPÄISCHE PATENTSCHRIFT

(45) Veröffentlichungstag der Patentschrift: **20.03.91**

(51) Int. Cl.5: **C07C 231/24**, C07C 233/36, C11D 1/90, A61K 7/08

(21) Anmeldenummer: **87103155.5**

(22) Anmeldetag: **05.03.87**

## (54) Verfahren zur Herstellung einer hochkonzentrierten, fliess- und pumpfähigen Betainlösung.

(30) Priorität: **24.04.86 DE 3613944**

(43) Veröffentlichungstag der Anmeldung:
**04.11.87 Patentblatt 87/45**

(45) Bekanntmachung des Hinweises auf die Patenterteilung:
**20.03.91 Patentblatt 91/12**

(84) Benannte Vertragsstaaten:
**BE CH DE ES FR GB IT LI NL**

(56) Entgegenhaltungen:
**DE-A- 2 557 456**
**US-A- 3 225 074**

(73) Patentinhaber: **Th. Goldschmidt AG**
**Goldschmidtstrasse 100 Postfach 101461**
**W-4300 Essen 1(DE)**

(72) Erfinder: **Bade, Volkbert, Dr.**
**Graffweg 38 b**
**W-4300 Essen 14(DE)**

EP 0 243 619 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft ein Verfahren zur Herstellung einer fließ- und pumpfähigen, mindestens 70 Gew.-% Betain der allgemeinen Formel

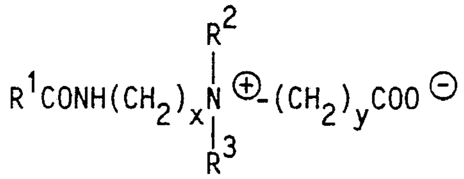

I

enthaltenden Lösung, wobei $R^1$ der Alkylrest einer Fettsäure oder eines Fettsäuregemisches mit im Mittel 6 bis 18 Kohlenstoffatomen ist, $R^2$, $R^3$ gleich oder verschieden sind und einen Alkyl- oder Hydroxyalkylrest mit 1 bis 4 Kohlenstoffatomen bedeuten, x einen Wert von 2 oder 3 und y einen Wert von 1, 2, 3 oder 4 haben, durch Quaternieren einer Verbindung der allgemeinen Formel $R^1CONH(CH_2)_xNR^2R^3$ mit einem Salz einer Halogencarbonsäure $X(CH_2)_yCOOMe$, wobei X ein Halogenrest und Me ein einwertiges Kation ist, bei erhöhten Temperaturen.

Betaine der obengenannten Formel haben in den vergangenen Jahren zunehmende Bedeutung zur Herstellung von Körperreinigungs- und -pflegemitteln erlangt. Sie verbinden ausgezeichnete Reinigungseigenschaften mit guter Hautverträglichkeit. Die Betaine bilden in wäßriger Lösung einen stabilen dichten Schaum aus, der auch in Gegenwart von Seife nicht zusammenfällt.

Die Herstellung solcher Betaine ist in vielen Patentschriften beschrieben, von denen die US-PS 3 225 074 stellvertretend genannt wird. Im allgemeinen setzt man dabei das entsprechende tertiäre Fettsäureamidamin mit dem Alkalisalz einer Halogencarbonsäure, in der Regel Chloressigsäure, um. Die Reaktion findet in wäßrigem Medium statt. Das bei der Reaktion entstehende Alkalichlorid wird in dieser wäßrigen Betainlösung belassen. Derartige Betaine werden deshalb im allgemeinen in einer Konzentration von etwa 30 Gew.-% in Form wäßriger oder wäßrig-alkalischer Lösungen in den Handel gebracht.

Es hat nicht an Versuchen gefehlt, höherkonzentrierte Lösungen der Betaine herzustellen, um den Transport und die Lagerung der Betainlösungen zu verbilligen.

Es ist dem Fachmann bekannt, daß mit steigender Konzentration einer wäßrigen Tensidlösung auch die Viskosität ansteigt. Häufig zeigt sich jedoch bei Überschreiten einer Konzentration von etwa 60 bis 70 Gew.-%, daß die Viskosität bei weiterer Konzentrationserhöhung auf ein Minimum abfällt, um dann erneut stark anzusteigen. Zur Erklärung dieser Viskositätsanomalie nimmt man an, daß sich in der Lösung eine Phase (sogenannte G-Phase) mit lamellarer Struktur ausbildet. Dieser Viskositätsabfall bei etwa 60 bis 70 Gew.-% Tensidlösung wird zur Herstellung hochkonzentrierter Tensidlösungen ausgenutzt und ist z.B. in "Soap, Perfumery, Cosmetics", 1982, 507 bis 509, beschrieben.

Diese Viskositätsanomalie bildet auch den Hintergrund für das Verfahren der DE-PS 25 57 456, bei dem zur Herstellung von Aminoxiden durch Oxidation von Aminen in Gegenwart von Wasser eine solche Wassermenge eingesetzt wird, daß im Produktgemisch die Wasserkonzentration 20 bis 30 Gew.-% beträgt. Bei diesem Verfahren wird somit bereits bei der Durchführung die Tensidkonzentration angestrebt, welche im Viskositätsminimum bei hohen Konzentrationen liegt.

Entzieht man den üblicherweise erhaltenen Betainlösungen das Wasser, steigt die Viskosität auch dieser Lösungen an. Die Lösungen werden jedoch bei etwa 40 Gew.-% Betain pastös und verfestigen sich bei weiterer Entwässerung zunehmend.

Eine Verflüssigung bei weiterer Trocknung des festen Produktes ist nicht zu beobachten. Es war deshalb nach dem Stand der Technik nicht möglich, hochkonzentrierte, mindestens 70 Gew.-% Betain der allgemeinen Formel I enthaltende Lösungen zu erhalten, die noch fließfähig und z.B. mit Pumpen dosierbar sind.

Der Erfindung liegt die Aufgabe zugrunde, fließ- und pumpfähige, mindestens 70 gew.-%ige Betainlösungen herzustellen, um damit die Verpackungs-, Transport- und Lagerhaltungskosten zu erniedrigen und die Compoundierung zu flüssigen Seifen, Shampoos, Duschgelen oder anderen kosmetischen Zubereitungen zu erleichtern. Der Erfindung liegt insbesondere die Aufgabe zugrunde, Betainlösungen mit mindestens 70 Gew.-% Betaingehalt herzustellen, die eine Viskosität $\leqq 25000$ mPas bei 25 °C aufweisen.

Überraschenderweise wurde gefunden, daß man diese Aufgabe von dem bekannten Verfahren des Standes der Technik ausgehend dadurch lösen kann, daß man

a) den Rest $R^1$ so auswählt, daß die Verbindung der Formel $R^1CONH(CH_2)_xNR^2R^3$ einen Schmelzpunkt

von höchstens 30 °C aufweist,

b) die Quaternierung

$b_1$) mit dem Kalium- oder Ammoniumsalz der Halogencarbonsäure $X(CH_2)_yCOOH$, wobei

$b_{11}$) das Salz in situ erzeugt werden kann,

$b_2$) in einem organischen polaren Lösungsmittel, welches höchstens 20 Gew.-% Wasser enthalten darf, durchführt,

c) nach der Quaternierung das gegebenenfalls enthaltene Wasser azeotrop abdestilliert und das ausgefallene Kalium- oder Ammoniumhalogenid abtrennt, danach

d) das Lösungsmittel ganz oder teilweise abdestilliert und

e) vor, gleichzeitig oder nach der Destillation die gewünschte Konzentration des Betains in dem anwendungstechnisch gewünschten Lösungsmittel oder Lösungsmittelgemisch einstellt.

Das Merkmal a) betrifft die Auswahl der geeigneten tertiären Amine für das erfindungsgemäß herzustellende Betain. Es hat sich gezeigt, daß nur dann fließ- und pumpfähige, höherkonzentrierte Betainlösungen erhalten werden, wenn der Schmelzpunkt der tertiären Amine der Formel $R^1CONH(CH_2)_xNR^2R^3$ höchstens 30 °C beträgt. Der Schmelzpunkt der tertiären Amine hängt dabei wesentlich von der Art der zur Herstellung der tertiären Amine verwendeten Fettsäure $R^1COOH$ oder dem entsprechenden Fettsäuregemisch ab. Je langkettiger die Fettsäure ist, um so höher ist der Schmelzpunkt des tertiären Amins. Verwendet man zur Herstellung des tertiären Amins der obigen Formel im wesentlichen reine, einheitliche Fettsäuren, sollte deren Kohlenstoffanzahl 10 nicht überschreiten. Bei Verwendung von Fettsäuregemischen ist eine Schmelzpunkterniedrigung zu beobachten. Man kann deshalb Fettsäuregemische einsetzen, deren mittlere Kettenlänge größer als die der reinen, einheitlichen Fettsäuren ist. So haben sich Fettsäuregemische als brauchbar erwiesen, die aus gehärtetem Kokosfett gewonnen werden und im Mittel etwa 12 Kohlenstoffatome aufweisen. Einfach und mehrfach ungesättigte Fettsäuren sind zur Herstellung der beim erfindungsgemäßen Verfahren zu verwendenden tertiären Amine ebenfalls geeignet.

Beispiele geeigneter Fettsäuren sind somit die gesättigten Fettsäuren mit 6 bis 10 Kohlenstoffatomen, z.B. $C_5H_{11}COOH$ (Kapronsäure), $C_7H_{15}COOH$ (Kaprylsäure), $C_9H_{19}COOH$ (Kaprinsäure); Gemische aus gesättigten Fettsäuren mit im Mittel bis zu 12 Kohlenstoffatomen; ungesättigte Fettsäuren, wie Ölsäure, Linolsäure, Ricinusölfettsäure oder deren Gemische mit gesättigten Fettsäuren.

Das Merkmal b) betrifft die Quaternierungsreaktion. Die Quaternierung erfolgt mit dem Kalium- oder Ammoniumsalz der Halogencarbonsäure $X(CH_2)_yCOOH$. Dabei kann das Kalium- oder Ammoniumsalz in situ erzeugt werden. Die Quaternierung erfolgt in einem organischen polaren Lösungsmittel. Es ist dabei von wesentlicher Bedeutung, daß das Lösungsmittel höchstens 20 Gew.-% Wasser enthalten darf.

Als organisches polares Lösungsmittel eignet sich besonders aliphatischer Alkohol mit 2 bis 4 Kohlenstoffatomen, wie Ethanol, Propanol und Butanol, insbesondere Isopropanol.

Entsprechend dem Merkmal c) des erfindungsgemäßen Verfahrens wird nach der Quaternierung das gegebenenfalls enthaltene Wasser azeotrop abdestilliert. Das ausgefallene Ammonium- oder Kaliumhalogenid wird aus dem Reaktionsprodukt entfernt. Dies kann zweckmäßig durch Filtration oder Zentrifugieren erfolgen.

Man erhält eine Lösung des gewünschten Betains in dem organischen polaren Lösungsmittel, welche jedoch noch gewisse restliche Mengen Ammonium-oder Kaliumhalogenid enthalten kann. Eine Abtrennung der Restmenge des Salzes, die etwa 2 bis 5 Gew.-% je nach verwendetem Lösungsmittel betragen kann, ist nicht erforderlich.

Um die so erhaltene Lösung des Betains der Formel I in dem verwendeten organischen polaren Lösungsmittel in eine Lösung überzuführen, die das anwendungstechnisch gewünschte Lösungsmittel oder Lösungsmittelgemisch enthält, wird nun das organische polare Lösungsmittel abdestilliert und vor, gleichzeitig oder nach der Destillation die gewünschte Konzentration des Betains in dem anwendungstechnisch gewünschten Lösungsmittel oder Lösungsmittelgemisch eingestellt. Soll die angestrebte, anwendungstechnisch gewünschte Lösung geringe Mengen des bei der Umsetzung verwendeten Alkohols enthalten, wird das Lösungsmittel nur in der darüber hinausgehenden Menge abdestilliert. Anwendungstechnisch gewünschte Lösungsmittel sind insbesondere aliphatische Diole mit 2 bis 4 Kohlenstoffatomen, wie Ethylenglykol, Propylenglykol oder Butylenglykol. Besonders bevorzugt ist Propylenglykol. Vorzugsweise setzt man die gewünschte Menge Diol der Lösung des Betains in dem organischen polaren Lösungsmittel zu und destilliert das organische polare Lösungsmittel ab. Man erhält eine Lösung des Betains in dem gewünschten Lösungsmittel, wobei sich die Konzentration des Betains entsprechend der zugesetzten Menge Lösungsmittel, wie z.B. Propylenglykol, einstellt.

Eine weitere Korrektur der Viskosität der erhaltenen Betainlösung kann dadurch erfolgen, daß man der so erhaltenen Lösung aliphatische Alkohole mit 2 bis 4 Kohlenstoffatomen, vorzugsweise Ethanol, und gegebenenfalls geringe Mengen Wasser zufügt. Der Zusatz des Alkohols kann in Mengen von 0,5 bis 10

Gew.-%, der Zusatz des Wassers in Mengen bis zu 5 Gew.-%, bezogen auf Gesamtgewicht der Zubereitung, erfolgen. Ist dieser Alkohol mit dem als Reaktionsmedium gewählten Alkohol identisch, wird die Menge in der Verfahrensstufe d) berücksichtigt.

Der Zusatz an aliphatischem Diol, Alkohol und gegebenenfalls Wasser erfolgt zweckmäßig bis zu einer Endkonzentration von

| | | |
|---|---|---|
| 70 | bis 97,5 | Gew.-% Betain |
| 2 | bis 15 | Gew.-% aliphatisches Diol |
| 0,5 | bis 10 | Gew.-% Ethanol |
| 0 | bis 5 | Gew.-% Wasser. |

Es war in Kenntnis des Standes der Technik nicht vorhersehbar gewesen, daß bei Einhaltung der Bedingungen des erfindungsgemäßen Verfahrens fließ- und pumpfähige, hochkonzentrierte Betainlösungen hergestellt werden können. Diese konzentrierten Lösungen sind in besonders wirtschaftlicher Weise abzufüllen, zu transportieren und zu verarbeiten.

Ein weiterer Gegenstand der Erfindung sind die konzentrierten Lösungen der Betaine. Diese haben folgende Zusammensetzung

| | | |
|---|---|---|
| 70 | bis 97,5 | Gew.-% Betain |
| 2 | bis 15 | Gew.-% aliphatischer Dialkohol mit 2 bis 4 Kohlenstoffatomen |
| 0,5 | bis 10 | Gew.-% aliphatischer Monoalkohol mit 2 bis 4 Kohlenstoffatomen |
| 0 | bis 5 | Gew.-% Wasser. |

Besonders bevorzugt sind Lösungen der Zusammensetzung

| | | |
|---|---|---|
| 70 | bis 97,5 | Gew.-% Betain |
| 2 | bis 15 | Gew.-% Propylenglykol |
| 0,5 | bis 10 | Gew.-% Ethanol |
| 0 | bis 5 | Gew.-% Wasser. |

In den folgenden Beispielen wird das erfindungsgemäße Verfahren noch näher erläutert.

Beispiel 1

600 g des Dimethylaminopropylamides von gehärteter Kokosölfettsäure, 220 g Chloressigsäure und 800 g Isopropanol werden in einen 2-l-Glaskolben gegeben, der mit einem Rührer, Thermometer, Rückflußkühler und Gaseinleitungsrohr ausgerüstet ist.

Das Reaktionsgemisch wird langsam unter Rühren auf 95 bis 98 °C erwärmt. Gleichzeitig wird Ammoniakgas in einer Menge von 10 bis 15 l/h durch das Gaseinleitungsrohr eingeleitet. Nach ca. 60 Minuten tritt durch Ausfällen des Ammoniumchlorids eine Eintrübung auf. Nach einer Gesamtreaktionszeit von ca. 4 Stunden wird die $NH_3$-Gaszugabe auf < 5 l/h gedrosselt und die Reaktion so lange weitergeführt, bis der Gehalt des eingesetzten Ausgangsamids unter 3 Gew.-% gefallen ist. Danach wird das Reaktionsgemisch auf Raumtemperatur abgekühlt und das ausgefallene Ammoniumchlorid abfiltriert. Es werden 78,9 g trockenes Ammoniumchlorid erhalten, entsprechend 65 % der theoretisch möglichen Menge.

Das Filtrat von 1574 g hat einen Feststoffgehalt von 49,6 Gew.-% und wird als Produkt A bezeichnet.

1574 g des Produktes A werden mit 138 g 1.2-Propylenglykol versetzt und anschließend vom

Isopropanol durch Vakuumdestillation befreit. Nach dem Abkühlen auf 50° C wird das Reaktionsprodukt (918,5 g) mit 19 g Ethanol versetzt und auf 30 bis 40° C abgekühlt. Bei dieser Temperatur werden 4 g $NaClO_2$ (15 %ig in Wasser) zur Bleichung zugegeben.

Das Endprodukt hat bei Raumtemperatur eine Viskosität von 20 000 mPas, eine Farbzahl nach Hess-Ives von 2 und die folgende Zusammensetzung: Betain 78,8 Gew.-%, Ammoniumchlorid 4,6 Gew.-%, Propylenglykol 14,6 Gew.-%, Ethanol 2,0 Gew.-%.

Beispiel 2

1000 g des Produktes A von Beispiel 1 werden mit 71,7 g 1.2-Propylenglykol versetzt. Isopropanol wird abdestilliert, danach dem Rückstand 30 g Ethanol zugegeben. Das erhaltene Produkt hat eine Viskosität von ca. 10 000 mPas, eine Farbzahl nach Hess-Ives von 2 und folgende Zusammensetzung: Betain 78,4 Gew.-%, Ammoniumchlorid 4,6 Gew.-%, Propylenglykol 12 Gew.-%, Ethanol 5 Gew.-%.

Beispiel 3

Abweichend von Beispiel 2 werden zusätzlich zum Ethanol noch 9 g Wasser zugegeben. Das erhaltene Produkt hat eine Viskosität von 5000 mPas und einen Wassergehalt von 3 Gew.-%. Der Betaingehalt der Lösung beträgt 75,4 Gew.-%.

Beispiel 4

1000 g des Produktes A werden mit 73 g Wasser und 26 g 1.2-Propylenglykol versetzt und gemäß Beispiel 1 unter Zugabe von 28 g Ethanol aufgearbeitet. Das erhaltene Produkt hat eine Viskosität von 900 mPas und einen Betaingehalt von 85 Gew.-%.

Beispiel 5

103 g Monochloressigsäure und 1015 g Isopropanol werden in einen 2-l-Glaskolben gegeben, der mit einem Rührer, Thermometer und Rückflußkühler ausgerüstet ist. Das Gemisch wird langsam unter Rühren auf 85° C erwärmt. Dann werden 73 g KOH-Plätzchen (85 %ig) zugesetzt. Es wird so lange die Temperatur bei 85° C gehalten, bis ein homogener Kristallbrei entstanden ist. Danach werden 300 ml Isopropanol zur Entfernung des Neutralisationswassers abdestilliert. Sodann werden 300 g des Dimethylaminopropylamides der gehärteten Kokosölfettsäure zugesetzt. Nach 8 h Reaktion unter Rückfluß ist der Gehalt des eingesetzten Ausgangsamides unter 10 Gew.-%, bezogen auf das gebildete Betain, gesunken. Das Reaktionsgemisch wird auf Raumtemperatur abgekühlt und das ausgefallene Kaliumchlorid abfiltriert. Es werden 75 g trockenes Kaliumchlorid erhalten, entsprechend 96 % der theoretisch möglichen Menge. Das Filtrat von 1094 g hat einen Feststoffgehalt von 35 Gew.-%.

1094 g des Filtrates werden mit 21 g 1.2-Propylenglykol versetzt und anschließend vom Isopropanol durch Vakuumdestillation befreit. Nach dem Abkühlen auf 50° C wird das Reaktionsprodukt (404 g) mit 21 g Ethanol versetzt und auf 30 bis 40° C abgekühlt.

Das Endprodukt hat bei Raumtemperatur eine Viskosität von 5000 mPas und die folgende Zusammensetzung: Betain 88 Gew.-%, Kaliumchlorid 1 Gew.-%, Wasser 1 Gew.-%, 1.2-Propylenglykol 5 Gew.-%, Ethanol 5 Gew.-%.

Beispiel 6

103 g Monochloressigsäure, 300 g des Dimethylaminopropylamides von gehärteter Kokosölfettsäure und 400 g Isopropanol werden in einen 2-l-Glaskolben gegeben, der mit einem Rührer, Thermometer, Rückflußkühler und Tropftrichter ausgerüstet ist. Das Reaktionsgemisch wird langsam unter Rühren auf 85 bis 90° C erwärmt. Es werden 124 g 50 %ige wäßrige Kalilauge innerhalb 1 h bei dieser Temperatur zugetropft. Danach beträgt der Restgehalt des eingesetzten Amidamines unter 10 Gew.-%, bezogen auf das entstandene Betain.

Nach dem Abkühlen wird mit 300 g Isopropanol verdünnt und das ausgefallene Kaliumchlorid abfiltriert. Es werden 78 g trockenes Kaliumchlorid erhalten, entsprechend 95 % der theoretisch möglichen Menge. Das Filtrat von 1149 g hat einen Feststoffgehalt von 33,7 Gew.-%.

1149 g des Filtrates werden mit 21,5 g 1.2-Propylenglykol versetzt und anschließend vom Isopropanol durch Vakuumdestillation befreit. Nach dem Abkühlen auf 50 °C wird das Reaktionsprodukt (408 g) zusätzlich mit 21,5 g Ethanol versetzt und auf 30 bis 40 °C abgekühlt.

Das Endprodukt hat bei Raumtemperatur eine Viskosität von 5000 mPas und die folgende Zusammensetzung: Betain 88 Gew.-%, Kaliumchlorid 1 Gew.-%, Wasser 1 Gew.-%, 1.2-Propylenglykol 5 Gew.-%, Ethanol 5 Gew.-%.

Die folgende Tabelle zeigt die Zusammensetzung und die Viskosität der Lösungen der Beispiele 1 bis 6.

Tabelle

| Zusammensetzung | Beispiel 1 | Beispiel 2 | Beispiel 3 | Beispiel 4 | Beispiel 5 | Beispiel 6 |
|---|---|---|---|---|---|---|
| Betain | 78,8 % | 78,4 % | 75,4 % | 85 % | 88 % | 88 % |
| $NH_4Cl$ | 4,6 % | 4,6 % | 4,6 % | 5 % | - | - |
| KCl | - | - | - | - | 1 % | 1 % |
| Propylenglykol | 14,6 % | 12 % | 12 % | 4,7 % | 5 % | 5 % |
| Ethanol | 2 % | 5 % | 5 % | 5 % | 5 % | 5 % |
| Wasser | - | - | 3 % | 0,9 % | 1 % | 1 % |
| Viskosität mPas | 20 000 | 10 000 | 5000 | 900 | 5000 | 5000 |

## Ansprüche

1. Verfahren zur Herstellung einer fließ- und pumpfähigen, mindestens 70 Gew.-% Betain der allgemeinen Formel

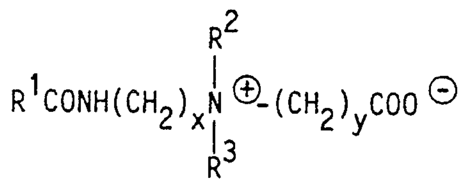

enthaltenden Lösung, wobei $R^1$ der Alkylrest einer Fettsäure oder eines Fettsäuregemisches mit im Mittel 6 bis 18 Kohlenstoffatomen ist, $R^2$, $R^3$ gleich oder verschieden sind und einen Alkyl- oder Hydroxyalkylrest mit 1 bis 4 Kohlenstoffatomen bedeuten, x einen Wert von 2 oder 3 und y einen Wert von 1, 2, 3 oder 4 haben, durch Quaternieren einer Verbindung der allgemeinen Formel $R^1CONH(CH_2)_xNR^2R^3$ mit dem Salz einer Halogencarbonsäure $X(CH_2)_yCOOMe$, wobei X ein Halogenrest und Me ein einwertiges Kation ist, bei erhöhten Temperaturen, dadurch gekennzeichnet, daß man
   a) den Rest $R^1$ so auswählt, daß die Verbindung der Formel $R^1CONH(CH_2)_xNR^2R^3$ einen Schmelzpunkt von höchstens 30 °C aufweist,
   b) die Quaternierung
      $b_1$) mit dem Kalium- oder Ammoniumsalz der Halogencarbonsäure $X(CH_2)_yCOOH$, wobei
      $b_{11}$) das Salz in situ erzeugt werden kann,
      $b_2$) in einem organischen polaren Lösungsmittel, welches höchstens 20 Gew.-% Wasser enthalten darf, durchführt,
   c) nach der Quaternierung das gegebenenfalls enthaltene Wasser azeotrop abdestilliert und das ausgefallene Kalium- oder Ammoniumhalogenid abtrennt, danach
   d) das Lösungsmittel ganz oder teilweise abdestilliert und
   e) vor, gleichzeitig oder nach der Destillation die gewünschte Konzentration des Betains in dem anwendungstechnisch gewünschten Lösungsmittel oder Lösungsmittelgemisch einstellt.

2. Verfahren nach Anspruch 1, dadurch gekennzeichnet, daß man die Quaternierung in einem aliphatischen Alkohol mit 2 bis 4 Kohlenstoffatomen durchführt.

3. Verfahren nach Anspruch 2, dadurch gekennzeichnet, daß man die Quaternierung in Isopropanol durchführt.

4. Verfahren nach einem oder mehreren der vorhergehenden Ansprüche, dadurch gekennzeichnet, daß man das Betain gemäß Merkmal e) in einem aliphatischen Dialkohol mit 2 bis 4 Kohlenstoffatomen löst.

5. Verfahren nach Anspruch 4, dadurch gekennzeichnet, daß man das Betain in Propylenglykol löst.

6. Verfahren nach Anspruch 4, dadurch gekennzeichnet, daß man das Betain in einem Gemisch aus einem aliphatischen Dialkohol und einem aliphatischen Monoalkohol mit 2 bis 4 Kohlenstoffatomen löst.

7. Verfahren nach Anspruch 6, dadurch gekennzeichnet, daß man das Betain in einem Gemisch aus einem aliphatischen Dialkohol und Ethanol löst.

8. Fließ- und pumpfähige, Betain der allgemeinen Formel des Patentanspruchs 1 enthaltende Lösung der Zusammensetzung

| | | | |
|---|---|---|---|
| 70 | bis 97,5 | Gew.-% | Betain |
| 2 | bis 15 | Gew.-% | aliphatischer Dialkohol mit 2 bis 4 Kohlenstoffatomen |
| 0,5 | bis 10 | Gew.-% | aliphatischer Monoalkohol mit 2 bis 4 Kohlenstoffatomen |
| 0 | bis 5 | Gew.-% | Wasser. |

**9.** Fließ- und pumpfähige, Betain der allgemeinen Formel des Patentanspruchs 1 enthaltende Lösung der Zusammensetzung

| | | | |
|---|---|---|---|
| 70 | bis 97,5 | Gew.-% | Betain |
| 2 | bis 15 | Gew.-% | Propylenglykol |
| 0,5 | bis 10 | Gew.-% | Ethanol |
| 0 | bis 5 | Gew.-% | Wasser. |

## Claims

**1.** Process for the preparation of a flowable and pumpable solution containing at least 70% by weight of the betaine of the general formula,

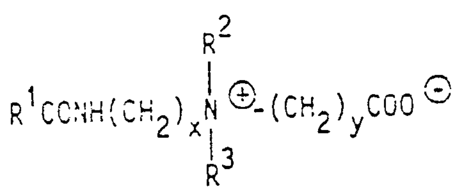

in which $R^1$ is the alkyl radical of a fatty acid or a mixture of fatty acids having on average 6 to 18 carbon atoms, $R^2$, $R^3$ are identical or different and are an alkyl or hydroxyalkyl radical having 1 to 4 carbon atoms, x has a value of 2 or 3 and y a value of 1, 2, 3 or 4, by quaternization of a compound of the general formula $R^1CONH(CH_2)_xNR^2R^3$ with the salt of a halogenocarboxylic acid $X(CH_2)_yCOOMe$, in which X is a halogen radical and Me a monovalent cation, at elevated temperatures, characterized in that

a) the radical $R^1$ is selected such that the compound of the formula $R^1CONH(CH_2)_xNR^2R^3$ has a melting point of at most 30 °C,

b) the quaternization is carried out

$b_1$) with the potassium salt or ammonium salt of the halogenocarboxylic acid $X(CH_2)_yCOOH$, in which

$b_{11}$) the salt can be produced in situ,

$b_2$) in an organic polar solvent which may contain at most 20% by weight of water,

c) any water present after the quaternization is distilled off azeotropically and the precipitated potassium halide or ammonium halide is separated off, then

d) the solvent is distilled off completely or in part and

e) the desired concentration of the betaine in the solvent or the solvent mixture desired for practical application is adjusted before, simultaneously with or after the distillation.

2. Process according to Claim 1, characterized in that the quaternization is carried out in an aliphatic alcohol having 2 to 4 carbon atoms.

3. Process according to Claim 2, characterized in that the quaternization is carried out in isopropanol.

4. Process according to one or more of the preceding claims, characterized in that the betaine is dissolved in accordance with feature e) in an aliphatic dialcohol having 2 to 4 carbon atoms.

5. Process according to Claim 4, characterized in that the betaine is dissolved in propylene glycol.

6. Process according to Claim 4, characterized in that the betaine is dissolved in a mixture comprising an aliphatic dialcohol and an aliphatic monoalcohol having 2 to 4 carbon atoms.

7. Process according to Claim 6, characterized in that the betaine is dissolved in a mixture comprising an aliphatic dialcohol and ethanol.

8. Flowable and pumpable solution containing the betaine of the general formula of Patent Claim 1 and having the composition

| | |
|---|---|
| 70 to 97.5 | % by weight of betaine |
| 2 to 15 | % by weight of aliphatic dialcohol having 2 to 4 carbon atoms |
| 0.5 to 10 | % by weight of aliphatic monoalcohol having 2 to 4 carbon atoms |
| 0 to 5 | % by weight of water. |

9. Flowable and pumpable solution containing the betaine of the general formula of Patent Claim 1 and having the composition

| | |
|---|---|
| 70 to 97.5 | % by weight of betaine |
| 2 to 15 | % by weight of propylene glycol |
| 0.5 to 10 | % by weight of ethanol |
| 0 to 5 | % by weight of water. |

## Revendications

1. Procédé de préparation d'une solution fluide et pouvant être pompée, contenant au moins 70 % en poids de bétaïne, répondant à la formule générale :

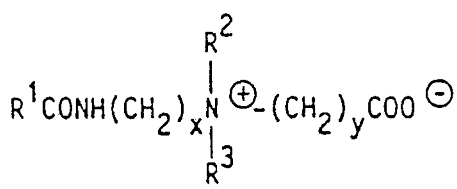

dans laquelle $R^1$ représente le radical alkyle d'un acide gras ou d'un mélange d'acides gras ayant en moyenne de 6 à 18 atomes de carbone, $R^2$ et $R^3$, qui sont identiques ou différents, représentent un radical alkyle ou hydroxyalkyle ayant de 1 à 4 atomes de carbone, x vaut 2 ou 3 et y vaut 1, 2, 3 ou 4,

par quaternisation d'un composé répondant à la formule générale $R^1CONH(CH_2)_xNR^2R^3$ avec le sel d'un hydracide halogéné de la formule $X(CH_2)_yCOOMe$ dans laquelle X représente un radical halogène et Me un cation monovalent, à des températures élevées, caractérisé en ce

a) qu'on choisit le radical $R^1$ de manière telle que le composé de formule $R^1CONH(CH_2)_xNR^2R^3$ présente un point de fusion de 30 °C au maximum,

b) on réalise la quaternisation

$b_1$) avec le sel de potassium ou d'ammonium de l'hydracide halogéné $X(CH_2)_yCOOH$,

$b_{1.1}$) le sel pouvant être produit in situ,

$b_2$) dans un solvant organique polaire qui peut contenir au maximum 20 % en poids d'eau,

c) on sépare par distillation azéotrope, après la quaternisation, l'eau éventuellement contenue et on sépare l'halogénure de potassium ou d'ammonium précipité, puis

d) on chasse le solvant par distillation, complètement ou partiellement et,

e) avant, pendant ou après la distillation, on règle la concentration de bétaïne souhaitée dans le solvant ou dans le mélange de solvants souhaité pour l'application technique.

**2.** Procédé selon la revendication 1, caractérisé en ce qu'on réalise la quaternisation dans un alcool aliphatique ayant de 2 à 4 atomes de carbone.

**3.** Procédé selon la revendication 2, caractérisé en ce qu'on réalise la quaternisation dans de l'isopropanol.

**4.** Procédé selon une ou plusieurs des revendications précédentes, caractérisé en ce qu'on dissout la bétaïne selon le point e) dans un diol aliphatique ayant de 2 à 4 atomes de carbone.

**5.** Procédé selon la revendication 4, caractérisé en ce qu'on dissout la bétaïne dans du propylène glycol.

**6.** Procédé selon la revendication 4, caractérisé en ce qu'on dissornt la bétaïne dans un mélange composé d'un diol aliphatique et d'un monoalcool aliphatique ayant de de 2 à 4 atomes de carbone.

**7.** Procédé selon la revendication 6, caractérisé en ce qu'on dissout la bétaïne dans un mélange composé d'un diol aliphatique et d'éthanol.

**8.** Solution fluide et capable d'être pompée, contenant une bétaïne répondant à la formule générale de la revendication 1, ayant la composition suivante :

70,0 à 97,5 % en poids de bétaïne
2,0 à 15,0 % en poids d'un diol aliphatique ayant de 2 à 4 atomes de carbone
0,5 à 10,0 % en poids d'un monoalcool aliphatique ayant de 2 à 4 atomes de carbone
0 à 5,0 % en poids d'eau.

**9.** Solution fluide et capable d'être pompée, contenant une bétaïne répondant à la formule générale de la revendication 1, ayant la composition suivante :

70,0 à 97,5 % en poids de bétaïne
2,0 à 15,0 % en poids de propylène glycol
0,5 à 10,0 % en poids d'éthanol
0 à 5,0 % en poids d'eau.